# EUROPEAN PATENT APPLICATION

(11) **EP 3 928 693 A1**
(43) Date of publication of application: **29.12.2021**
(21) Application number: 20758479.8
(22) Date of filing: 13.02.2020
(51) Int. Cl.: A61B 5/053, A61B 5/00

(54) **MEASUREMENT DEVICE USING EIT ELECTRODE**

(30) Priority: 21.02.2019 KR 20190020522
(71) Applicant: Bilab Co., Ltd., Gyeonggi-do 13486 (KR)
(72) Inventor: WI, Hun, Suwon-si Gyeonggi-do 16712 (KR); CHANG, Jun, Seoul 08014 (KR)
(74) Representative: Michalski Hüttermann & Partner Patentanwälte mbB
(86) International application number: PCT/KR2020/002014
(87) International publication number: WO 2020/171473

(57) **Abstract**

A body fat measurement device, disclosed according to the present invention, comprises: at least one electrode unit which supports at least one EIT electrode and is tightly attachable to skin; and a sensor unit which measures electrical impedance tomography information by sensing a signal measured from the electrode unit, wherein, after being connected to the sensor unit and used once, the electrode unit may be separated from the sensor unit and replaced. According to such configuration, the one-time used electrode unit can be replaced from the sensor unit, and thus economic efficiency can be achieved, and convenience of use can be increased.

## Description

### TECHNICAL FIELD

The present invention relates to a measurement device using EIT electrode, and more particularly, to a measuring device using EIT (Electrical Impedance Tomography) electrodes, in which one-time use of the electrodes can improve economic feasibility, convenience of use, and measurement accuracy.

### BACKGROUND ART

Bioimpedance test is a method of examining body composition with values of electrical signal changes (bioimpedance values) by cell membrane and tissue, by injecting microcurrent through electrodes attached to the body and measuring voltages, and is mainly used to examine health conditions inside the body such as muscle, fat, cell, body mass, basic metabolite, in-body moisture, metabolic activity, and bone mineral, plasma etc.

EIT (Electrical Impedance Tomography) is used for performing imagination of physiological functions such as respiration and blood flow by attaching a plurality of electrodes to the body and repeating current injection and voltage measurement, and then restoring the impedance distribution image for the cross section of the body.

In this EIT test, a subj ect wears a vest or belt-type device equipped with a plurality of EIT electrodes and the voltages of the subject are measured. At this time, since the device provided with the EIT electrodes was used multiple times, there was a risk of bacterial infection, including sweat and body odor of other subjects. In addition, when the device is to be applied to a seriously injured patient, it is difficult to attach a plurality of EIT electrodes to the patient.

Accordingly, various studies that can increase the convenience, reliability, etc. of electrical impedance tomography of a subject using the EIT electrode are steadily being conducted in recent years.

### DETAILED DESCRIPTION OF THE INVENTION

### TECHNICAL PROBLEM

It is an object of the present invention to provide a measurement device using EIT electrode that is economical and has improved measurement convenience by using disposable electrode (replaceable after one(single)-time use).

It is another object of the present invention to provide a measurement device using EIT electrode that can measure various parts and can change configuration of the electrode unit according to measurement conditions, thereby improving measurement accuracy.

### SUMMARY OF THE INVENTION

In order to achieve the above objects, a measurement device using disposable EIT electrode according to the present invention comprises at least one electrode unit configured to support at least one of EIT electrodes to be in close contact with skin; and a sensor unit configured to sense signal measured by the electrode unit to measure electrical impedance image information.

In addition, the electrode unit comprises at least one pad type electrode supporter which supports the at least one of EIT electrodes and is extended to a longitudinal direction so as to be in close contact with skin. Wherein the electrode supporter comprises a electrode line for electrical connection with the EIT electrode.

In addition, at least one end of the electrode supporter can be configured to be connected to the sensor unit to electrically connect the EIT electrode and the sensor unit each other.

In addition, an adhesive medium configured for attaching the electrode supporter to skin is provided on one side of the electrode supporter on which the at least one of EIT electrode is supported. The adhesive medium can be configured to have at least one expose hole through which the at least one of EIT electrodes is exposed to contact skin.

In addition, a plurality of the EIT electrodes are configured to be provided in a sticker type having adhesive strength and are individually attached to skin; and each of the plurality of EIT electrodes can be configured to be connected to the sensor unit through each of the connection lines.

In addition, the electrode unit comprises at least one electrode supporter connectable to the sensor unit and at least one electrode line extending from the electrode supporter to electrically connect the electrode supporter and the at least one EIT electrode, respectively.

In addition, the at least one electrode line can be configured to have different lengths extending from the electrode supporter.

In addition, the at least one EIT electrode is configured to be provided with an indicator on which an identification color or picture is formed, the electrode unit can be configured to be provided with a barcode or a RFID (radio frequency identification) tag for providing information on the EIT electrode.

In addition, at least one sensor unit is configured to be provided, and a plurality of the electrode units can be configured to be provided to be simultaneously connected to the at least one sensor unit.

In addition, the sensor unit is configured to be connected to a reference electrode that is adhered to skin and provide a reference of the electrode potential measured by the electrode unit, and the reference electrode is mounted on the sensor unit, so that the sensor unit can be attached to skin with an adhesive strength of the reference electrode.

In addition, the sensor unit is configured to be connected to a reference electrode that provides a reference of the electrode potential measured by the electrode unit, the sensor unit can be configured to be disposed near the electrode unit and the reference electrode so as to be connected to the reference electrode through an electrode line.

In addition, the plurality of EIT electrodes can be configured to be provided, and any one of the plurality of EIT electrodes can be configured to be provided For reference electrode for providing a reference of the electrode potential measured by the electrode unit, and can be configured to be connected to the sensor unit.

### EFFECT OF THE INVENTION

In accordance with the present invention having the above configuration, firstly, by replacing the disposable electrode unit after one-time use for the sensor unit, existing problems such as bacterial infection can be improved by one-time use of the electrode unit, and thus the reliability can be improved for the subject.
Secondly, a relatively inexpensive electrode unit is used once at a time, thereby being economical.
Third, the electrode unit can be replaced in response to various measurement conditions, thereby being possible to secure measurement diversity and improve measurement accuracy.
Fourth, a user can easily replace and disconnect the electrode unit from the sensor unit, and thus, the user's convenience is excellent.
Fifth, it is easy to change the attachment position of the electrode unit, so that it is possible to provide various electrical impedance tomography information by sharing a single sensor unit.

### BRIEF DESCRIPTION OF THE DRAWINGS

FIG. 1 is a schematic plan view of a measurement device using EIT electrode according to a first preferred embodiment of the present invention.
FIG. 2 is an exploded perspective view schematically disassembled of the electrode unit shown in FIG. 1.
FIG. 3 is a plan view schematically illustrating a top view, a side view, and a bottom view of the electrode unit shown in FIG. 2.
FIG. 4 is a plan view schematically illustrating a modification of the electrode unit shown in FIG. 2.
FIG. 5 is an exploded perspective view schematically enlarged and disassembled of the sensor unit shown in FIG. 1.
FIG. 6 is a view schematically illustrating a state in which a reference electrode is provided on the other surface of the sensor connector shown in FIG. 5.
FIG. 7 shows views schematically illustrating a state in which an electrode unit and a sensor unit are attached to skin of a subject.
FIG. 8 is a diagram schematically illustrating a state in which a measurement device using EIT electrode according to a second preferred embodiment of the present invention is attached to a subject.
FIG. 9 is a plan view schematically illustrating various modifications of the electrode unit shown in FIG. 8.
FIG. 10 is a plan view schematically showing a state in which the electrode unit shown in FIG. 9 is connected to the electrode protector.
FIG. 11 is a plan view schematically illustrating an electrode unit of a measurement device using EIT electrodes according to a third preferred embodiment of the present invention.
FIG. 12 is a diagram schematically illustrating a state in which the electrode shown in FIG. 11 is attached to skin of a subject.
FIG. 13 is a plan view schematically illustrating an electrode unit of a measurement device using EIT electrodes according to a fourth preferred embodiment of the present invention.

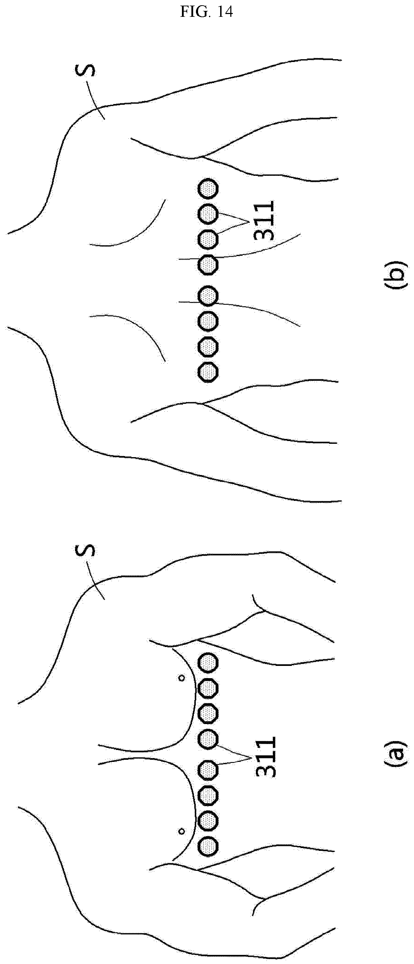

FIG. 14 is a view schematically illustrating a state in which the electrode shown in FIG. 13 is attached to skin of a subject.

### EMBODIMENTS OF THE INVENTION

Hereinafter, preferred embodiments of the present invention will be described with reference to the accompanying drawings. However, the idea of the present invention is not limited to such embodiments, and the idea of the present invention may be differently proposed by adding, changing, and deleting components constituting the embodiments, but these ideas are also included in the scope of the present invention.

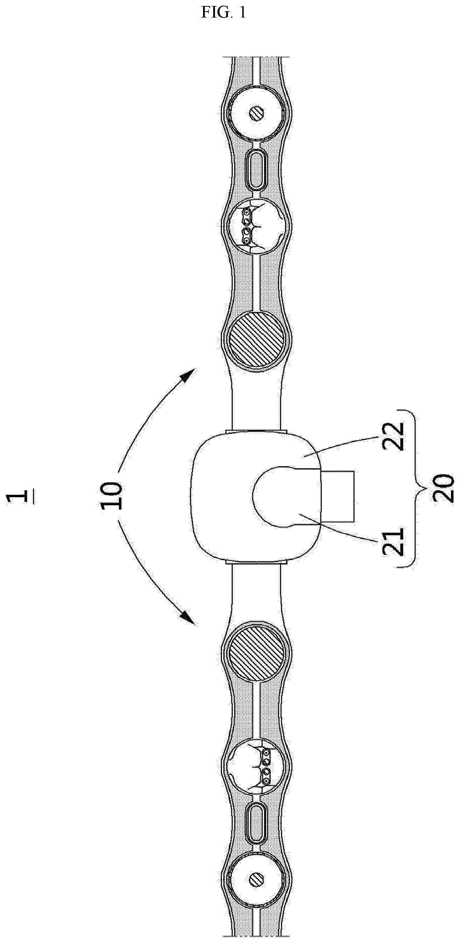

Referring to FIG. 1, a measurement device 1 using EIT electrodes according to a first preferred embodiment of the present invention comprises an electrode unit 10 and a sensor unit 20.

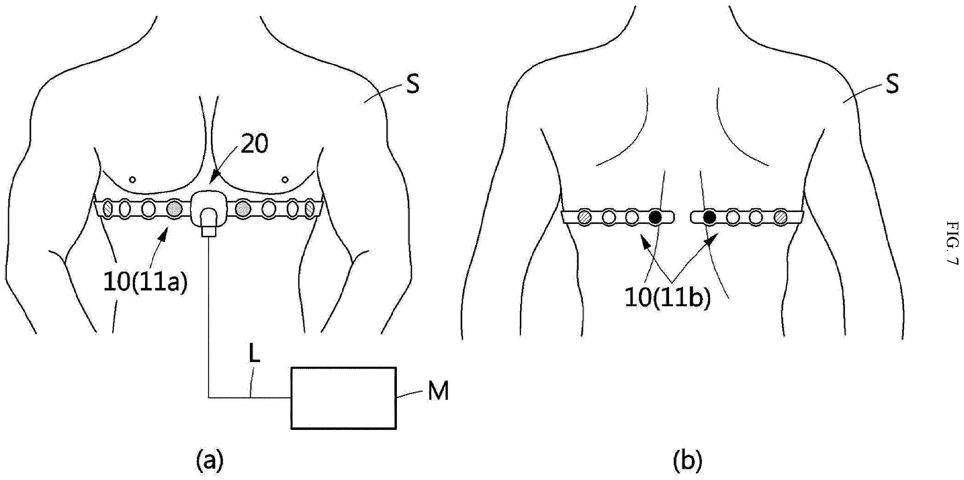

For reference, the measurement device 1 using EIT electrode described in the present embodiment is shown and described as being attached to the subject's skin S (see FIG. 7) and measuring to monitor pulmonary respiration (lung respiration) and cardiac output. However, the present invention is not limited thereto, and the measurement device 1 using EIT electrode can be modified to measure and monitor not only pulmonary respiration and cardiac output, but also various biometric information such as body fat, body cells, water content, bone minerals, plasma, and basal metabolic rate.

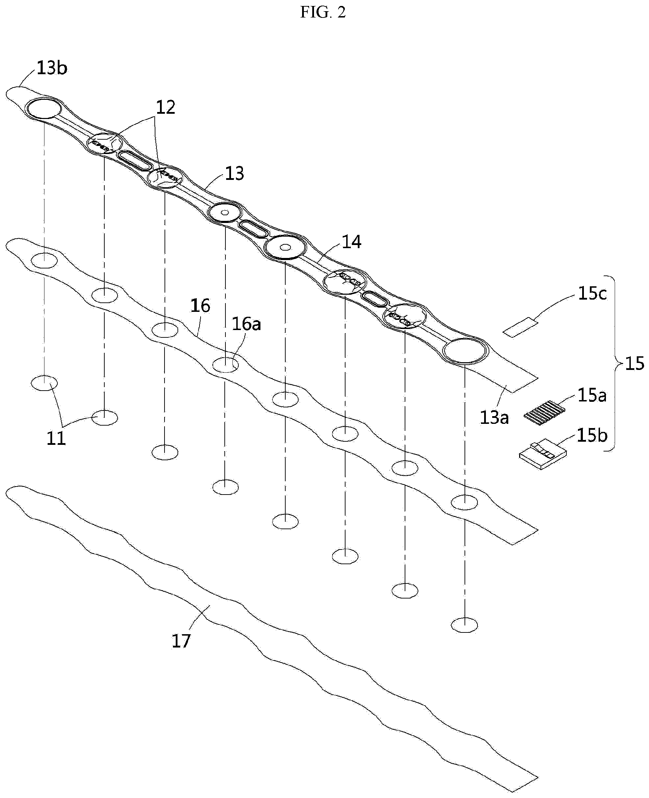

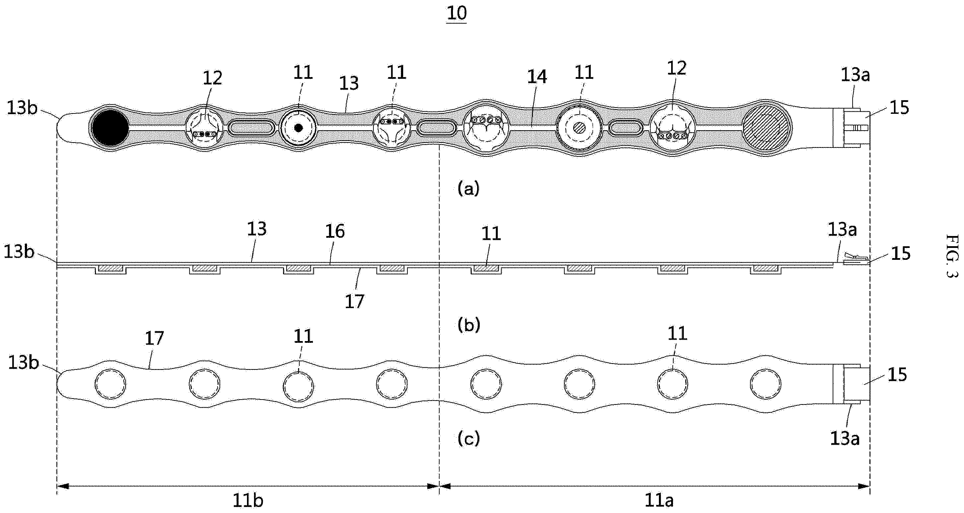

The electrode unit 10 comprises at least one EIT electrode 11 capable of being in close contact with skin S. As shown in FIGS. 2 and 3, the electrode unit 10 comprises EIT electrodes 11 and an electrode supporter 13. In FIG. 3, (a) is a top view of the electrode unit 10, (b) is a side view of the electrode unit 10, and (c) is a bottom view of the electrode unit 10.

As shown in FIG. 2, a plurality of the EIT electrodes 11 can be provided and arranged in a line to be spaced apart from each other in a longitudinal direction. However, the embodiment is not limited to the illustration of FIG. 2, but it is understood that the EIT electrodes 11 can be provided with a single or multiple columns and rows spaced apart from each other.

The electrode supporter 13 supports at least one EIT electrode 11, extends in the longitudinal direction, and has a thin band shape so as to be in close contact with skin S. In addition, one EIT electrode 11 or a plurality of EIT electrodes 11 are provided on the electrode supporter 13 to be spaced apart from each other, and an electrode line 14 for electrical connection with the EIT electrode 11 can be formed on the electrode supporter 13.

For reference, it is possible to improve the quality of measurement data in the EIT tomography method by embedding the electrode line 14 in the electrode supporter 13 supporting the EIT electrode 11 so that the electronic circuit is located near the EIT electrode 11.

On the other hand, at least one electrode supporter 13 is provided and connected to the sensor unit 20 attached to skin S of the subject, and then is separated from the sensor unit 20 and discarded after use.

In addition, an indicator 12 for user identification is provided in the plurality of EIT electrodes 11. The indicator 12 can be formed of an identifiable image (picture) or color so as to indicate the position to which the EIT electrode 11 is applied or the position of the embedded built-in EIT electrode 11. Although the indicator 12 is illustrated as being provided in plurality corresponding to each of the plurality of EIT electrodes 11, it can be provided to only some of the plurality of EIT electrodes 11.

In the present embodiment, a plurality of EIT electrodes 11 are supported on a single electrode supporter 13, and the EIT electrodes 11 can be distinguished from a first electrode region 11a attached to the front side of the subject and a second electrode region 11b attached to the rear side of the subject. Although the first and second electrode regions 11a and 11b are provided by four in each, and totally eight EIT electrodes are exemplified to be supported by the electrode supporter 13, it is understood that the number of the EIT electrodes 11 is not limited as shown in FIG. 2.

The electrode supporter 13 shown in FIGS. 2 and 3 is provided in at least one pair, and one end 13a of each electrode supporter 13 is connected to the sensor unit 20. In addition, the other end 13b of the electrode supporter 13 is in close contact with skin S along the circumference of the subject (see FIG. 7). For this reason, a total of 16 EIT electrodes 11 are attached to the subject to apply current to the subject in a 16-channel manner. In this case, an inserter 15 inserted into the sensor unit 20 and electrically connectable can be provided at one end 13a of the electrode supporter 13. The inserter 15 is electrically connected to each of the plurality of EIT electrodes 11 through the electrode lines 14 and comprises a connector 15a connected to the sensor unit 20, and the connector 15a is supported by an insertion housing 15b and is protected by a reinforcing member 15c.

On the other hand, as shown in FIG. 2, the first and second electrode regions 11a and 11b have two or more different colors and pictures, thereby guiding the identification of the user. For example, the EIT electrodes 11 of the first electrode region 11a provided at one end 13a of the electrode supporter 13 are provided with a blue indicator 12, and the EIT electrodes 11 of the second electrode region 11b provided on the other end 13b of the electrode supporter 13 are provided with a red indicator 12, and thereby guiding the identification of one end 13a connected to the sensor unit 20. In addition, as illustrated as being attached along the body of the subject according to the exemplary embodiment of the present invention, the first electrode region 11a to be attached to the front side of the subject can be provided with the indicator 12 of the front side picture of the body, and the second electrode area 11b attached to the rear side of the subject can be provided with the indicator 12 of the rear side picture of the body.

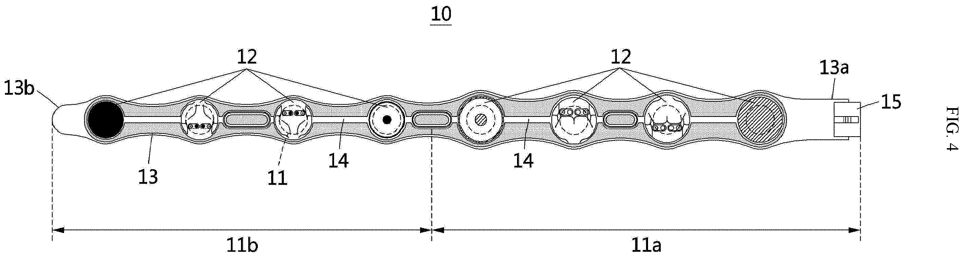

As described above, the EIT electrodes 11 of the first and second electrode regions 11a and 11b provide identifications to the user by the identifiers 12 having at least more than two colors and pictures, and the colors and pictures of the indicators 12 can be variously changed as shown in FIG. 4 according to the parts and conditions to which each of the EIT electrodes 11 is applied.

In addition, the electrode supporter 13 can be provided as a sticker having a self-adhesive strength or a pad which can be closely attached to skin S. In the present embodiment, as shown in FIGS. 2 and 3, it is illustrated that the electrode supporter 13 has a pad shape in which the adhesive medium 16 is stacked, thereby being attached to skin S.

The adhesive medium 16 has a band shape extending in the longitudinal direction to correspond to the electrode supporter 13 and forms exposure holes 16a for exposing each of the EIT electrodes 11 supported on the electrode supporter 13 to the outside. In addition, as shown in (c) of FIG. 3, it is preferable that a protective film 17 is attached on the lower surface of the electrode unit 10, that is, on the adhesive surface of the adhesive medium 16, for the protection of the adhesive strength and the EIT electrodes 11 before being attached to skin S of the subject.

As described above, in the electrode unit 10, a plurality of EIT electrodes 11 are supported on the electrode supporter 13, the plurality of EIT electrodes 11 are exposed through the exposure holes 16a of the adhesive medium 16 and are protected by the protective film 17. Thereinafter, when attached to the subject's skin S, the protective film 17 is separated and removed from the adhesive medium 16 and is in close contact with skin S, in the state that the plurality of EIT electrodes 11 are supported in the electrode supporter 13 by the adhesive strength of the adhesive medium 16.

Meanwhile, although not shown in detail, the electrode unit 10 can be provided with a barcode or RFID (Radio Frequency Identification) tag capable of providing each of information, for example, whether genuine or not, a measurement part, a measurement target, and the like. The barcode or RFID tag of the electrode unit 10 can provide information through electrical signal exchange with an external electronic device.

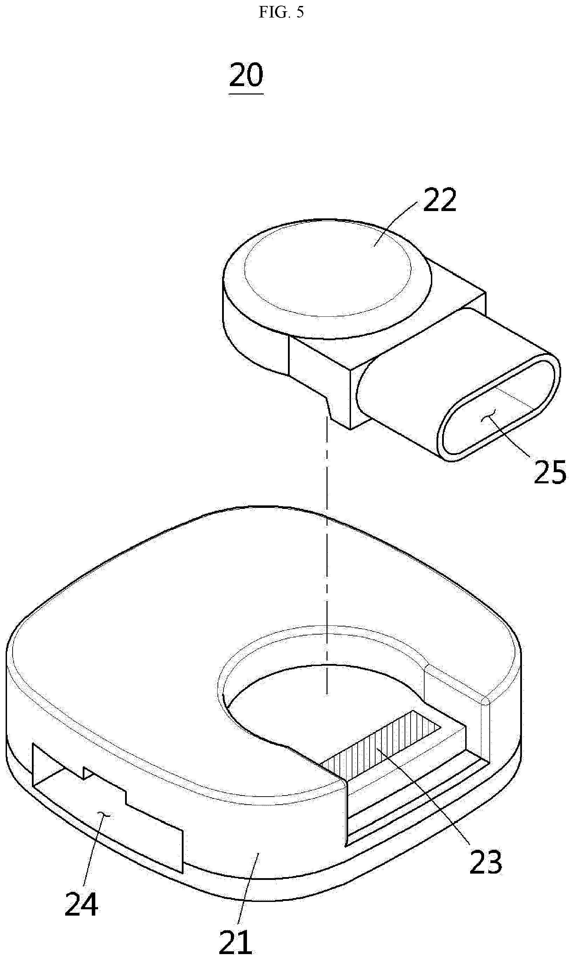

The sensor unit 20 is selectively connected to the electrode unit 10 to sense a signal measured from the connected electrode unit 10 to measure a body composition such as pulmonary respiration and cardiac output of a subject. As shown in FIG. 5, the sensor unit 20 comprises a sensor 21 and a sensor connector 22.

The sensor 21 senses a signal received from the electrode unit 10. Such a sensor 21 senses the pulmonary respiration and cardiac output of a subject by injecting current into a pair of the EIT electrodes 11 of the electrode unit (10) and measuring the impedance from the other the EIT electrodes 11 excepting for the pair of EIT electrodes 11.

The sensor connector 22 interconnects the external electronic device and the sensor 21 by electrically connecting to the connection port 23 provided on the sensor 21. The sensor connector 22 is provided with a connection port 25 for signal exchange with an electronic device (M) (see FIG. 7) such as a display device like an external monitor or a control device. For reference, not only electrical signal exchange with the outside is possible through the connection port 25, but also power can be supplied to the sensor 21 through the connection port 25.

On the other hand, an insertion hole 24 to which the inserter 15 of the electrode unit 10 is inserted and connected is provided at both sides of the sensor 21. At this time, it is shown and illustrated as an example that the electrode unit 10 is connected to the sensor unit 20 by coupling the inserter 15 shown in FIG. 3 and FIG. 4 to the insertion hole 24 in a buckle manner, but the connection method of the electrode unit (10) to the sensor unit (20) can be variously modified.

The sensor unit 20 comprises an adhesive medium having an adhesive strength and can be connected to the electrode unit 10 after being attached to skin S of the subject. In the present embodiment, it is shown and illustrated as an example that a reference electrode 26 is provided in the sensor unit 20 for providing a reference of the electrode potential measured by the electrode unit 10, and the sensor 21 is attached to skin S by the adhesive strength of the reference electrode 26.

That is, in the present embodiment, the sensor unit 20 is attached to skin S using the adhesive strength of the reference electrode 26. When attached to skin S using the reference electrode 26, the sensor 21 of the sensor unit 20 is provided with a first snap 27, and the reference electrode 26 is provided with a second snap 28 corresponding to the first snap 27 so that the reference electrode 26 is mounted on the sensor 21 by the mutual combination of the first and second snaps 27 and 28. It should be understood that the coupling method of the sensor 21 and the reference electrode 26 is not limited to the above illustrated example.

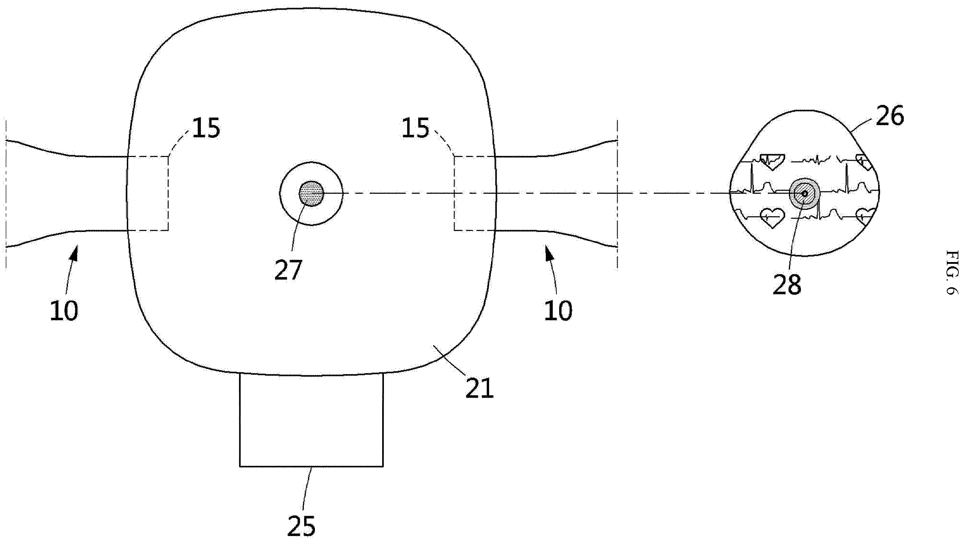

In addition, although FIG. 6 shows that the reference electrode 26 is integrally provided with respect to the sensor 21, the reference electrode 26 is not limited thereto. That is, although not shown in detail, the reference electrode 26 can be provided separately from the sensor 21 and attached to the subject's skin S at a position spaced apart from the sensor 21 so as to provide a reference of the electrode potential measured by the electrode unit 10. Moreover, a modified embodiment in which any one of the plurality of EIT electrodes 11 is provided for reference electrode and is connected to the sensor unit 20 is also possible.

When the sensor unit 20 does not use the adhesive strength of the reference electrode 26, the sensor unit 20 can be provided on an attachment surface attached to skin S with an adhesive strength such as double-sided tape, so that the sensor unit 20 can be attached to skin S. For reference, it is possible to modify the example that sensor unit 20 is provided with a separate adhesive medium along with the adhesive strength of the reference electrode 26, thereby being doubly attached to skin S. Also, it is apparent that the sensor unit 20 is not attached to skin S and is placed near the subject so that the sensor unit 20 is connected to the electrode unit 10.

An electrical impedance tomography operation of the measurement device 1 using EIT electrode according to the present invention having the above configuration will be described with reference to Fig. 7.

First, as shown in (a) of FIG. 7, the sensor unit 20 is positioned on skin S of the subject to be measured. In this embodiment, it is described that the sensor unit 20 is attached to the subject's skin S using the adhesive strength of the reference electrode 26.

In a state where the sensor unit 20 is placed on the subject's skin S, the electrode unit 10 is connected to the sensor unit 20. More specifically, the electrode supporters 13 supporting the eight EIT electrodes 11 shown in FIGS. 2 and 3 are provided as a pair, and one end 13a of each of the pair of electrode supporters 13 is inserted into the insertion hole 24 of the sensor 21, and thus the electrode unit 10 is connected to the sensor unit 20. In addition, as shown in (b) of FIG. 7, the other end 13b of each of the pair of electrode supporters 13 is in the state of being attached to skin S along the circumference of the subject.

In this case, the user identifies the plurality of EIT electrodes 11 by the image(picture) or color of the identifier 12 and connects the electrode unit 10 to the sensor unit 20. More specifically, the user attaches the first electrode region (or area) 11a on the front side of the subject and locates the second electrode region 11b on the rear side of the subject, and then the user connects the electrode unit 10 to the sensor unit 20.

For reference, in the electrode unit 10, the electrode supporter 13 is attached to skin S by the adhesive strength of the adhesive medium 16 from which the protective film 17 is separated, and the EIT electrodes 11 exposed through the exposure holes 16a of the adhesive medium 16 are in direct contact with skin S, so that current is injected to skin S through the EIT electrodes 11.

The electrode unit 10 is connected to the sensor unit 20 in this way, and then the electrode unit 10 can pass microcurrent into the body of the subject and the sensor unit 20 senses changes in electrical signals to measure biometric information such as pulmonary respiration and cardiac output of the subject. Such measured information is provided through an external line L to an external device M such as a monitor provided externally or the like.

On the other hand, when the measurement is completed, the electrode unit 10 is separated from the sensor unit 20 and discarded, so that the electrode unit 10 is just used once (i.e., disposable). Thereafter, when it is desired to measure the subject's pulmonary respiration and cardiac output again, a new electrode unit 10 is connected to the previously attached sensor unit 20 to measure the body composition, and then the electrode unit 10 is separated from the sensor unit 20 again and discarded. As described above, since only the electrode unit 10, which is relatively inexpensive compared to the sensor unit 20, is discarded after being used once, it is economical and has excellent applicability to various measurement conditions without bacterial infection.

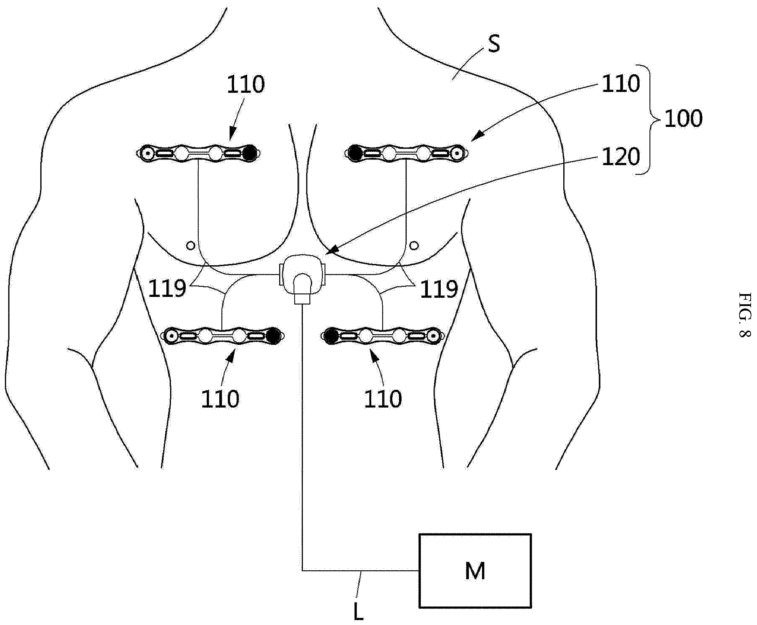

Referring to FIG. 8, a measurement device 100 using EIT electrode according to a second preferred embodiment of the present invention is schematically illustrated.

The measurement device 100 using EIT electrode according to the second embodiment comprises an electrode unit 110 and a sensor unit 120. Here, since the configuration of the sensor unit 120 is similar to that described in the first embodiment with references to FIGS. 1 through 7, a detailed description thereof is omitted.

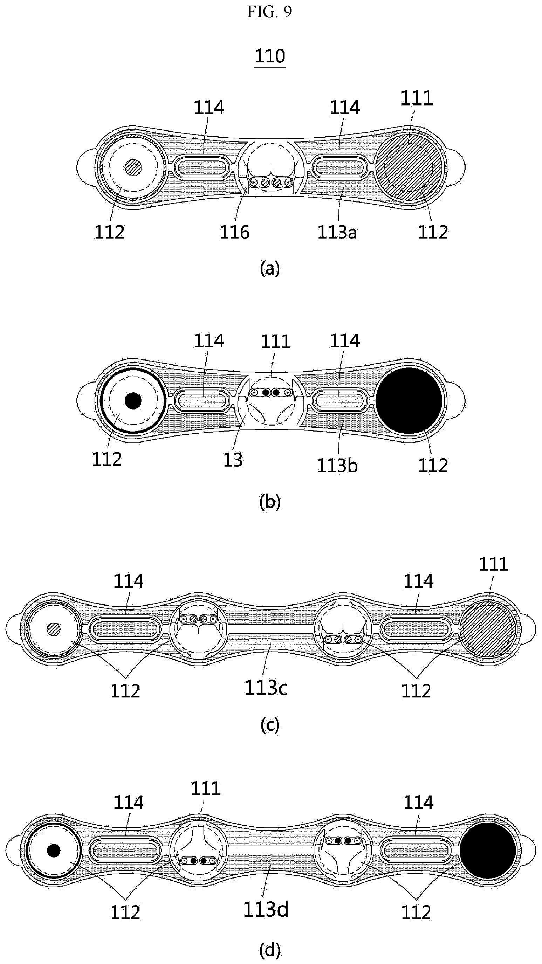

In the electrode unit 110 according to the second embodiment, as shown in FIG. 9, a plurality of EIT electrodes 111 are provided on electrode supporters 113a, 113b, 113c, and 113d. In this case, the electrode line 114 is provided on the electrode supporters 113 to electrically connect the plurality of EIT electrodes 111 to each other. Here, the plurality of EIT electrodes 111 can be identified by indicators 112.

In addition, the electrode supporters 113 have adhesive strength and are attached to skin S of the subject, so that the electrode unit 110 applies current to the subject in a kind of sticker electrode manner. For reference, the number of electrode unit 110 attached to the subject is not limited to the illustrated example.

A plurality of electrode units 110 such as stickers can be attached to the subject's skin S and can be simultaneously connected to a single sensor unit 120 through a connection line 119. At this time, the single sensor unit 120 is provided and connected to the plurality of electrode units 110 through the connection line 119 in a state in which the plurality of electrode units 110 are attached to the subject's skin S.

The sensor unit 120 senses an electric signal change value provided from the plurality of electrode units 110 and provides the measured electrical impedance tomography information to the external device M.

When the measurement of biometric information such as pulmonary respiration and cardiac output is completed by connecting the electrode unit 110 and the sensor unit 120, the plurality of electrode units 110 are separated from the sensor unit 120 and discarded.

Meanwhile, although the sensor unit 120 is not shown in detail, the sensor unit 120 can be connected to a reference electrode (not shown) and can be attached to skin S by sharing the adhesive strength of the reference electrode in the same manner as in the first embodiment. However, the sensor unit 120 can be connected to a reference electrode (not shown) using a separate lead line, and the reference electrode can be attached to skin S and the sensor unit 120 can be placed in an unattached state at a position connectable to the electrode unit 110.

Referring to FIG. 9, various embodiments of the electrode unit 110 are illustrated.

In FIG. 9, (a) shows an electrode supporter 113a having three EIT electrodes 111 attachable to the front side of the subject, and (b) shows an electrode supporter 113b that supports three EIT electrodes 111 attachable to the rear side of the subject. In addition, the electrode supporters 113c and 114d for supporting the four EIT electrodes 111 are respectively shown in FIG. 9 (c) and FIG. 9 (d). Here, the EIT electrodes 111 supported on the electrode supporters 113a and 113c shown in FIG. 9 (a) and FIG. 9 (c) are identified by the indicators 112 to be attached to the front side of the subject, the EIT electrodes 111 supported on the electrode supporters 113b and 113d shown in FIG. 9 (b) and FIG 9 (d) are identified by the indicators 112 to be attached to the rear side of the subject.

As such, the electrode supporters 113a, 113b, 113c, and 113d shown in FIG. 9 (a) to (d) are provided to be selectively connected to the sensor unit (120) by selecting the number of EIT electrodes 111 according to the conditions to be measured.

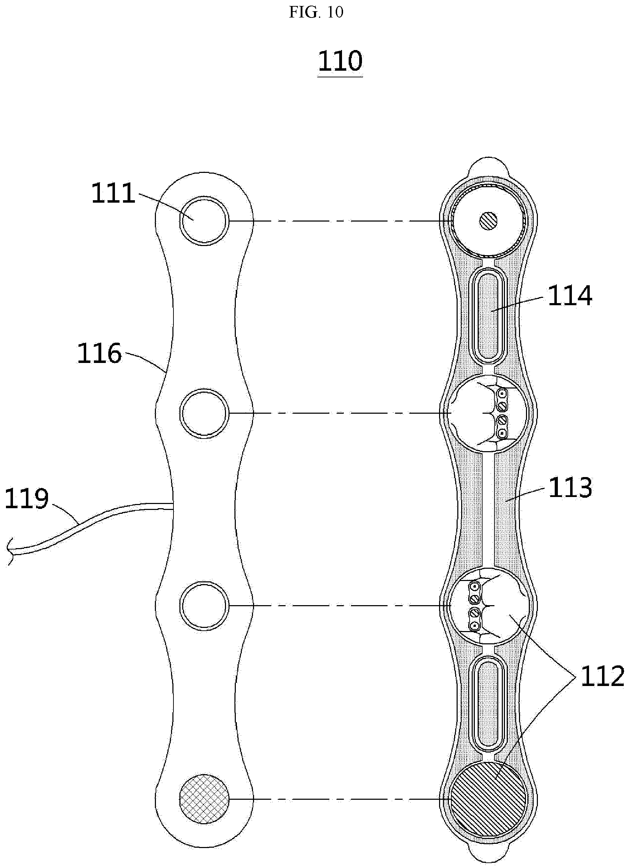

For reference, as shown in FIG. 10, the electrode supporter 113 of the electrode unit 110 is provided as a kind of thin pad on which the indicators 112 for indicating the EIT electrodes 111 and the electrode line 114 are provided, the electrode supporter 113 can be adhered to skin S in a state where the adhesive medium 116 is laminated on the electrode supporter 113 to expose the EIT electrodes 111. In this case, the adhesive medium 116 can be provided with a connection line 119 for electrical connection between the plurality of EIT electrodes 111 and the sensor unit 120, and the adhesive medium 116 can be protected by a protective film (not shown).

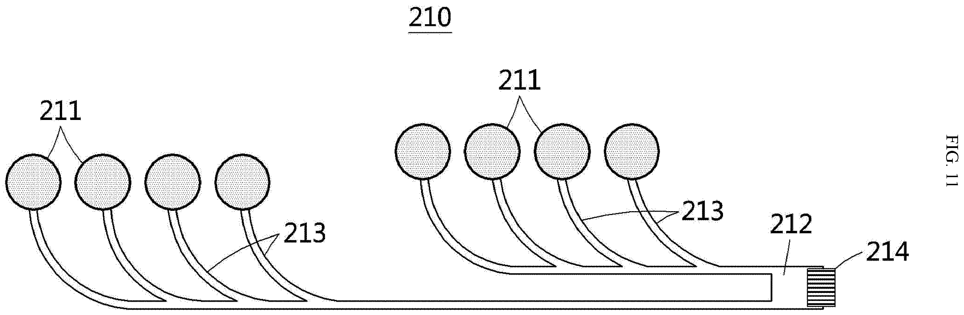

FIG. 11 schematically shows an electrode unit 210 of a measurement device using EIT electrode according to a third preferred embodiment of the present invention. For reference, since the configuration of the sensor unit of the measurement device using EIT electrode according to the third embodiment is similar to those described in the above-described first and second embodiments, a detailed description and illustration will be omitted.

As shown in FIG. 11, the electrode unit 210 according to the third embodiment comprises a plurality of EIT electrodes 211 and an electrode supporter 212 supporting the EIT electrodes 211. A plurality of EIT electrodes 211 are connected to the electrode supporter 212 through electrode lines 213. At this time, the electrode lines 213 are separated from the electrode supporter 212 and has a lead shape extending to the plurality of EIT electrodes 211, respectively. A connector 214 for electrical connection with the sensor unit (see FIG. 1) is provided at one end of the electrode supporter 212.

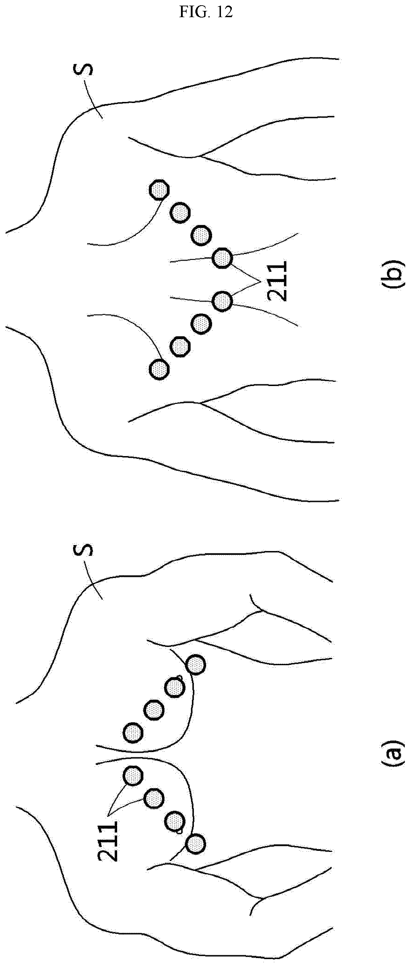

The electrode unit 210 having such a configuration according to the third embodiment of the present invention is provided in a sticker type in which each of the plurality of EIT electrodes 211 has self-adhesive strength. Thus, compared to the band-shaped electrode supporters 113 described above, the positions of the EIT electrodes 211 can be adjusted relatively freely with respect to the electrode supporter 212 as shown in FIG. 12. More specifically, so it is easy to change the attachment positions of the EIT electrodes 211 in the case that the EIT electrodes 211 are in close contact with skin S in a diagonal direction to measure the body composition as shown in FIG. 12, as well as in the case of the first and second embodiments in which a plurality of EIT electrodes 211 are arranged in a line in a horizontal direction to each other.

For reference, the sticker type electrode unit 210 according to the third embodiment is also connected to the sensor unit to be used once, separated from the sensor unit, and then discarded.

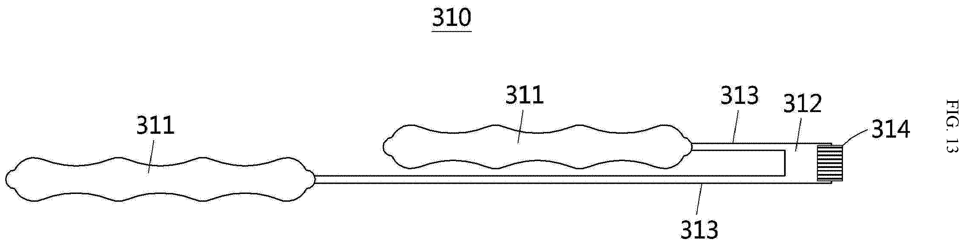

Referring to FIG. 13, an electrode unit 310 of a measurement device using EIT electrode according to a fourth embodiment of the present invention is schematically illustrated. For reference, since the configuration of the sensor unit (see FIG. 1) of the measurement device using the EIT electrodes according to the fourth embodiment is similar to that of the first and second embodiments described above, a detailed description and illustration are omitted.

The electrode unit 310 according to the fourth embodiment comprises a plurality of EIT electrodes 311 and an electrode supporter 312 supporting the plurality of EIT electrodes 311, and a connector 314 for electrical connection with the sensor unit (see FIG. 1) is provided at one end of the electrode supporter 312. Here, a plurality of EIT electrodes 311 are connected to the electrode supporter 312 through electrode lines 313, and the length of the electrode lines 313 is different from each other. That is, the electrode lines 313 are separated from the electrode supporter 312 and are connected to the plurality of EIT electrodes 311 with different lengths, and thus the plurality of EIT electrodes 311 are extended with different lengths from the sensor unit (not shown) and connected thereto.

According to the fourth embodiment, a plurality of EIT electrodes 311 can be provided in a sticker type so as to be attachable to skin S of the subject and can be attached to skin S of the subject.

For reference, as described in the fourth embodiment, the EIT electrodes 311 can be elliptical shape extending in the longitudinal direction, unlike the aforementioned circular shaped EIT electrodes. The shapes of the EIT electrode 311 are not limited to the first to fourth embodiments and can be provided in various shapes suitable for measurement conditions, such as a circle, an ellipse, and a polygon.

As described above, all the electrode units 10, 110, 210, and 310 shown in the first to fourth embodiments are provided so as to be able to variously change the position where they are attached to the subject, and the number of EIT electrodes 11, 111, 211, and 311 can also be changed according to the measurement conditions of the subject. Accordingly, it is possible to measure various body composition information as well as pulmonary respiration and cardiac output of the subject through electrical impedance tomography. In addition, since the electrode units 10, 110, and 210 are connected to the sensor units 20 and 120 and used once at a time, then separated and discarded, existing problems such as bacterial infection can also be improved.

As described above, although described with reference to the preferred embodiment of the present invention, it will be understood by those skilled in the art that various modifications and variations can be made in the present invention without departing from the spirit and scope of the present invention as set forth in the claims below.

## Claims

1. A measurement device using EIT electrode, comprising:
an electrode unit configured to support at least one of EIT electrodes to be in contact with skin; and
a sensor unit configured to sense signal measured by the electrode unit to obtain electrical impedance image information,
wherein the electrode unit is disposable, in that the electrode unit is configured to be connected to the sensor unit, used for once at a time, and detached from the sensor unit to be replaced.

2. The measurement device using EIT electrode of claim 1, wherein the electrode unit comprising:
at least one of electrode supporters configured to support the at least one of EIT electrodes and to be a pad type extending to a longitudinal direction so as to be in contact with skin;
wherein the at least one of the electrode supporters is configured to be provided with at least one of electrode lines for electrically connecting to the EIT electrodes.

3. The measurement device using EIT electrode of claim 2, wherein at least one end of each of the electrode supporters is configured to be connected to the sensor unit, and electrically interconnect the at least one of EIT electrodes and the sensor unit.

4. The measurement device using EIT electrode of claim 2, wherein the at least one side of each of the electrode supporters by which at least one of EIT electrodes is supported is configured to be provided with an adhesive medium configured to attach the electrode supporters to skin, and the adhesive medium is configured to form an exposure hole for contacting the EIT electrode to skin by exposing the at least one of the EIT electrodes through the exposure hole.

5. The measurement device using EIT electrode of claim 1, wherein each of EIT electrodes is configured to be provided in a sticker type with adhesive strength so as to be individually attached to skin, and
the EIT electrodes are configured to be connected to the sensor unit through each of the connection lines.

6. The measurement device using EIT electrode of claim 1, wherein the electrode unit comprising:
at least one electrode supporter configured to be connectable to the sensor unit; and
at least one electrode line configured to be extending from the electrode supporter, and electrically connecting the electrode supporter and the at least one of EIT electrodes, respectively.

7. The measurement device using EIT electrode of claim 6, wherein the at least one of electrode lines is configured to have different lengths extending from the electrode supporter.

8. The measurement device using EIT electrode of claim 1, wherein the at least one of EIT electrodes is configured to provided with an indicator formed with identification color or figure, and
the electrode unit is configured to be provided with a barcode or an RFID tag for providing the information of the EIT electrodes.

9. The measurement device using EIT electrode of claim 1, wherein a plurality of the electrode units are configured to be simultaneously connected to a single sensor unit.

10. The measurement device using EIT electrode of claim 1, wherein the sensor unit is configured to be connected to a reference electrode that is adhered to skin and provides a reference of an electrode potential measured by the electrode unit, and
the reference electrode is mounted on the sensor unit, and the sensor unit is attached to skin by the adhesive strength of the reference electrode.

11. The measurement device using EIT electrode of claim 1, wherein the sensor unit is configured to be connected to a reference electrode that provide a reference of an electrode potential measured by the electrode unit, and
the sensor unit is configured to be placed near the electrode unit and the reference electrode so as to be connectable using the reference electrode and the electrode line.

12. The measurement device using EIT electrode of claim 1, wherein the electrode unit is configured to provide a plurality of EIT electrodes, any one of the plurality of EIT electrodes is configured to be a reference electrode providing a reference of a electrode potential measured by the electrode unit, and connected to the sensor unit.
